# EUROPEAN PATENT SPECIFICATION

(11) **EP 3 685 780 B1**
(45) Date of publication and mention of the grant of the patent: **19.05.2021**
(21) Application number: 19184769.8
(22) Date of filing: 05.07.2019
(51) Int. Cl.: A61B 18/12, A61B 34/00, A61B 34/35, A61B 90/00, A61B 90/50

(54) **A ROBOT-ASSISTED SYSTEM FOR TRANSURETHRAL SURGERY**
ROBOTERGESTÜTZTES SYSTEM FÜR DIE TRANSURETHRALE CHIRURGIE
SYSTÈME ASSISTÉ PAR UN ROBOT POUR LA CHIRURGIE TRANSURÉTRALE

(30) Priority: 25.01.2019 CN 201910075428
(43) Date of publication of application: 29.07.2020
(73) Proprietor: Li, Hanzhong, Dongcheng District, Beijing 100730 (CN); Wang, Junchen, Haidian District, Beijing 100083 (CN); Zhang, Xuebin, Dongcheng District, Beijing 100730 (CN)
(72) Inventor: Li, Hanzhong, Dongcheng District, Beijing 100730 (CN); Wang, Junchen, Haidian District, Beijing 100083 (CN); Zhang, Xuebin, Dongcheng District, Beijing 100730 (CN)
(74) Representative: HGF

(56) References cited:
- EP-A1- 0 571 827
- CN-A- 106 667 580
- ES-A1- 2 200 679
- JP-B2- 3 476 878
- US-A1- 2017 056 094

## Description

### TECHNICAL FIELD

The present disclosure relates to robot technologies, and in particular, relates to a robot-assisted system for transurethral surgery using a resectoscope.

### BACKGROUND

The transurethral surgery is a minimally invasive surgery mainly for resection of benign prostatic hyperplasia and bladder tumor using a resectoscope.

At the present stage, in the transurethral surgery, a patient lies on a surgical table in a lithotomy position. A surgeon sits in front of the patient's perineum and manipulates the resectoscope for surgery.

During the operation, the surgeon needs to frequently adjust the position and orientation of the resectoscope to cut pathological tissues in different directions. Due to the limited field of view and the lack of global understanding towards the surgical site, the pathological tissues are prone to be left in situ (incomplete resection), and the irregular cutting path, may result in poor outcomes in terms of urination for the patient with prostatic hypertrophy. An inexperienced surgeon may be disorientated, damage the adjacent tissues or organs, which will cause risks. Meanwhile, the whole surgery also consumes a lot of labor. Further, since the position of the surgeon is adjacent to the patient, he or she is also susceptible to being contaminated by patient's body fluid.

ES 2200679A1 discloses a robot teleoperation system for transurethral resection of the prostate, where the robot teleoperation system includes a computer, a video screen, a controller, an end effector, endoscope and a robot arm, etc. US 2017/056094A1 discloses a resectoscope including a movable cutting electrode at the distal part of the device and discloses the use of this device in a teleoperation setup. JP 3476878B2 discloses a robotic system for transurethral surgery comprising a resectoscope with a cutting electrode, a robotic arm, a control apparatus and a display and the system is teleoperated using an operation rod. CN 106667580A discloses a resectoscope holder that can be fixed on a robotic arm to perform transurethral prostatectomy.

EP 0571827A1 discloses a teleoperated robotic system for controlling the position of a scoping device or a medical instrument. This system also includes an automatic mode in which an instrument is automatically repositioned based on a selected point in an image taken from a scoping device.

### SUMMARY

The present invention is defined by appended claim 1. Preferred embodiments are disclosed in the dependent claims.

Embodiments of the present application provide a robot-assisted system for transurethral surgery, so that the surgeon can perform surgical procedures remotely, implements the remote surgery and precise operation, thereby increasing the operation safety while saving the surgeon's labor.

An embodiment of the present application provides a robot-assisted system for transurethral surgery, including:
a display device, communicatively connected to a workstation and for receiving display data transmitted from the workstation and displaying images to a surgeon according to the display data;
a human-computer interaction device, communicatively connected to the workstation and for acquiring operation instructions input by the surgeon and transmitting the operation instructions to the workstation;
a resectoscope including an electrified wire loop (cutting loop), the resectoscope is communicatively connected to the workstation and for transmitting acquired image information to the workstation and controlling a current intensity of the electrified wire loop according to a first control signal received from the workstation;
a resectoscope holding device, communicatively connected to the workstation and for holding and controlling the resectoscope and driving the cutting loop to move forward and backward along an axis of the resectoscope according to a second control signal received from the workstation;
a six-degree-of-freedom series-connected mechanical arm, communicatively connected to the workstation and fixed to the resectoscope holding device, and for driving the resectoscope holding device to move forward and backward along the axis of the resectoscope, rotate around a central axis of the resectoscope and perform remote center motion (RCM) around a point on the central axis of the resectoscope according to a third control signal received from the workstation;
the workstation is used for generating the display data according to the image information acquired from the resectoscope and transmitting the display data to the display device; generating the first control signal according to the operation instructions acquired from the human-computer interaction device and transmitting the first control signal to the resectoscope, and/or generating the second control signal and transmitting the second control signal to the resectoscope holding device, and/or generating the third control signal and transmitting the third control signal to the six-degree-of-freedom series-connected mechanical arm.

The robot-assisted system for transurethral surgery provided by the embodiments of the present application enables the surgeon to perform surgical operations remotely, implements the remote surgery and precise operation, thereby increasing the operation safety while saving the labor of the surgeon.

### BRIEF DESCRIPTION OF DRAWINGS

FIG. 1 is a structural diagram of a robot-assisted system for transurethral surgery according to an embodiment of the present application;
FIG. 2 is a structural diagram of another robot-assisted system for transurethral surgery according to an embodiment of the present application;
FIG. 3 is a structural diagram of a resectoscope holding device according to an embodiment of the present application;
FIG. 4 is a structural diagram of yet another robot-assisted system for transurethral surgery according to an embodiment of the present application.

### DETAILED DESCRIPTION

Hereinafter the present application will be further described in detail in conjunction with the drawings and embodiments. It should be understood that the specific embodiments set forth below are merely intended to illustrate and not to limit the present application. Additionally, it should be noted that, for ease of description, only part, not all, of the structures related to the present application are illustrated in the drawings.

### Embodiments

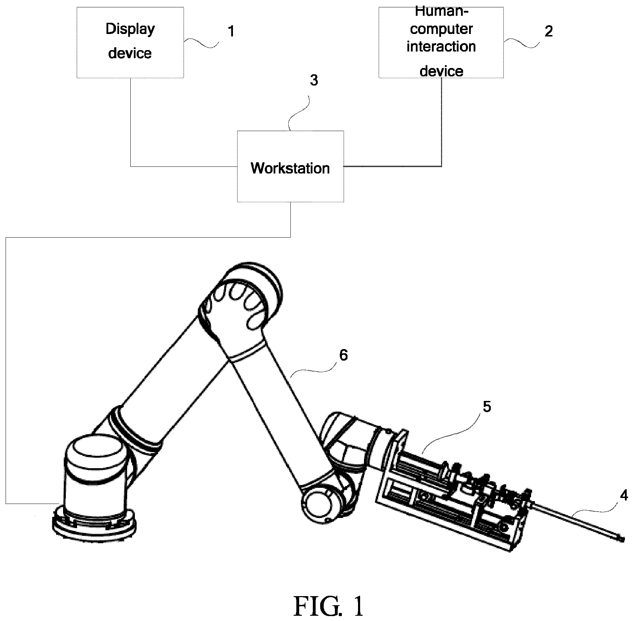

An embodiment of the present application provides a robot-assisted system for transurethral surgery. This embodiment may be applicable to the case where surgical operations are performed through a robot-assisted system for transurethral surgery, as shown in FIG. 1, the system includes a display device 1, a human-computer interaction device 2, a workstation 3, a resectoscope 4, a resectoscope holding device 5 and a six-degree-of-freedom series-connected mechanical arm 6.

The display device 1 is communicatively connected to the workstation 3, and is configured to receive display data transmitted from the workstation 3 and display images to a surgeon based on the display data. The display device 1 may be a liquid crystal display screen. When a surgeon performs the surgery by manipulating the robot-assisted system for transurethral surgery, the resectoscope 4 will be inserted into the affected part of the patient to acquire an image of the affected part, then the image is transmitted to the workstation 3, and image information acquired from the resectoscope 4 is transformed into display data recognizable by the display device 1 by the workstation 3, thereby displaying the image of the affected part to the surgeon.

The human-computer interaction device 2 is communicatively connected to the workstation 3, and is configured to acquire operation instructions input by the surgeon and transmit the operation instructions to the workstation 3. The human-computer interaction device 2 acquires the surgeon's operation for controlling a current intensity of an electrified wire loop (cutting loop) 41, and/or acquires the surgeon's operation for controlling the cutting loop 41 to move forward and backward, and/or acquires the surgeon's operation for controlling space movement of the resectoscope 4, for example, controlling the resectoscope 4 to move forward and backward along an axis of the resectoscope, rotate around a central axis of the resectoscope and rotate around a preset point on the central axis of the resectoscope.

The resectoscope 4 includes the cutting loop 41. The resectoscope is communicatively connected to the workstation 3, and is configured to transmit acquired image information to the workstation 3 and control the current intensity of the cutting loop 41 according to a first control signal received from the workstation 3. The surgeon controls resection speed and hemostasis efficiency by controlling the current intensity of the cutting loop 41.

The resectoscope holding device 5 is communicatively connected to the workstation 3, and is configured to hold the resectoscope 4 to drive the cutting loop 41 to move forward and backward along the axis of the resectoscope according to a second control signal received from the workstation 3. The resectoscope holding device 5 clamps and fixes the resectoscope 4, and may drive the cutting loop 41 to move forward and backward along the axis of the resectoscope 4, so that the cutting loop 41 accurately reaches a specified position to implement resection or hemostasis procedures.

The six-degree-of-freedom series-connected mechanical arm 6 is communicatively connected to the workstation 3 and fixed to the resectoscope holding device 5. The six-degree-of-freedom series-connected mechanical arm 6 is configured to drive the resectoscope holding device 5 to move forward and backward along the axis of the resectoscope, rotate around a central axis of the resectoscope and rotate around the preset point on the central axis of the resectoscope according to a third control signal received from the workstation 3. The six-degree-of-freedom series-connected mechanical arm 6 is provided with six joints so as to drive the resectoscope holding device 5 to move forward and backward along the axis of the resectoscope, rotate around a central axis of the resectoscope and rotate around the preset point on the central axis of the resectoscope, ensuring flexibility of various actions during the surgery.

The workstation 3 is configured to: generate display data according to the image information acquired from the resectoscope 4 and transmit the display data to the display device 1; generate the first control signal according to the operation instructions acquired from the human-computer interaction device 2 and transmit the first control signal to the resectoscope 4; and/or generate the second control signal and transmit the second control signal to the resectoscope holding device 5; and/or generate the third control signal and transmit the third control signal to the six-degree-of-freedom series-connected mechanical arm 6.

The workstation 3 is a core control device of the robot-assisted system for transurethral surgery, and generally is a computer device with a data processing function. It is to be understood that various modules of the robot-assisted system for transurethral surgery may be connected in a wired or wireless manner. In FIG. 1, connection lines between the resectoscope holding device 5 and the workstation 3 are not shown, and connection lines between the six-degree-of-freedom series-connected mechanical arm 6 and the workstation 3 are also not shown.

The robot-assisted system for transurethral surgery provided by the embodiment of the present application enables the surgeon to perform surgical operations remotely, implements the remote surgery and precise operation, thereby increasing the operation safety while saving the labor of the surgeon.

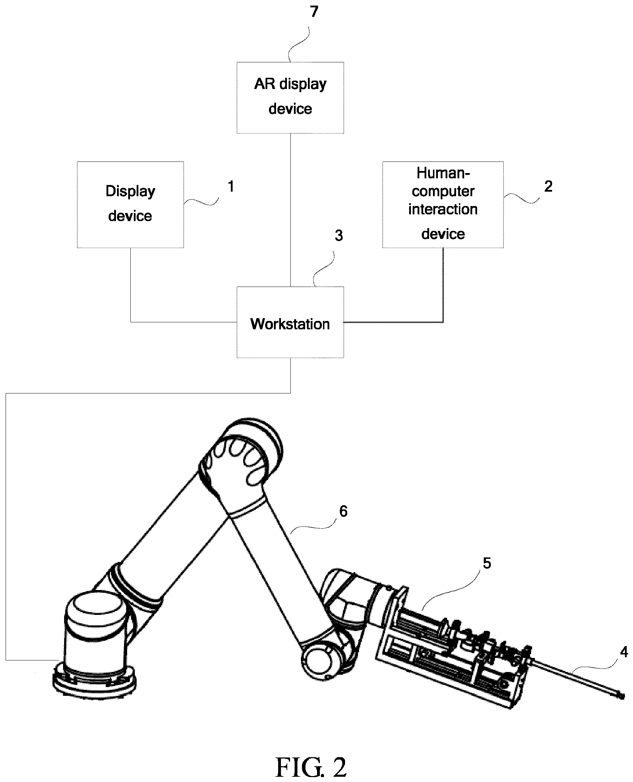

On the basis of the above technical solutions, as shown in FIG. 2, the robot-assisted system for transurethral surgery may further include an Augmented Reality (AR) display device 7.

The AR display device 7 is communicatively connected to the workstation 3, and is configured to display 3D images to the surgeon according to AR display data acquired from the workstation 3.

The workstation 3 is further configured to generate the AR display data according to the image information acquired from the resectoscope 4 and transmit the AR display data to the AR display device.

Through the AR display device 7, the surgeon may observe the 3D image showing the internal condition of the patient, which is beneficial to observe the affected part for improving the accuracy of surgical actions.

Optionally, the human-computer interaction device 2 includes a three-degree-of-freedom operating rod or a three-degree-of-freedom mouse, six first function keys and a second function key.

The six first function keys and six joints of the six-degree-of-freedom series-connected mechanical arm are in a one-to-one correspondence so that a control mode of the joint corresponding to the preset function key is activated or exited according to input operations of the surgeon. That is, when the surgeon presses one of the six first function keys, if it is not currently in a control mode for a joint corresponding to the first function key, then it is switched to the control mode for the joint, in this case, if the surgeon manipulates the three-degree-of-freedom operating rod to rotate around a preset axis, actions of the joint will be adjusted correspondingly. When the surgeon presses one of the first function keys, if it is currently in the control mode for a joint corresponding to the first function key, then the control mode for the joint is exited.

The second function key is used for acquiring a key travel signal generated by pressing the second function key by the surgeon so that the workstation 3 generates the first control signal according to the key travel signal. The second function key is a key with a preset key travel. With different key travel depth the surgeon presses, different key travel signal will be generated. The key travel signal is transmitted to the workstation 3, the workstation 3 generates the corresponding first control signal according to the key travel signal to control the current intensity of the cutting loop 41. Exemplarily, with the increasing of the depth of the second function key pressed by the surgeon, the current intensity of the cutting loop 41 correspondingly increases. The workstation 3 may read the state of an operation rod and keys of the human-computer interaction device 2 by a USB port. The control data obtained through processing the state is used for controlling the six-degree-of-freedom series-connected mechanical arm 6 and the resectoscope holding device 5. Meanwhile, the workstation 3 may receive position data of various joints of the six-degree-of- freedom series-connected mechanical arm 6 and position data of a component driving the cutting loop 41 to move in the resectoscope holding device 5 for the subsequent processing.

Optionally, on the basis of the above technical solutions, the robot-assisted system for transurethral surgery may further include a force sensor.

The force sensor is communicatively connected to the workstation 3 and disposed at the junction of the resectoscope holding device 5 and the six-degree-of-freedom series-connected mechanical 6arm. The force sensor is configured to measure a contacting force between the resectoscope 5 and an object which is in contact with the resectoscope, and transmit a signal about the contacting force to the workstation 3.

Correspondingly, the workstation 3 is further configured to control the resectoscope 4, the resectoscope holding device 5 and the six-degree-of-freedom series-connected mechanical arm 6 to stop working when the signal about the contacting force acquired from the force sensor is greater than a preset value. In the whole surgery process, the contacting force between the resectoscope 4 and the patient may be acquired by the force sensor. When the contacting force is too large, the surgeon may immediately stop the robot and then adjust the robot to an appropriate surgical posture.

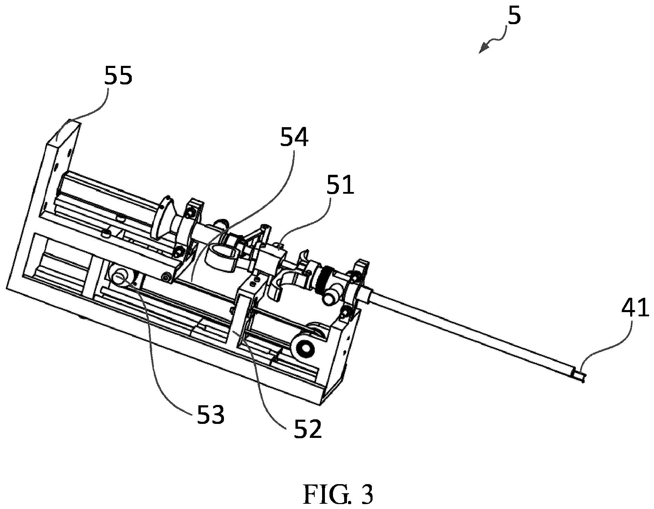

As shown in FIG. 3, optionally, the resectoscope holding device 5 is screwed to a flange at an end joint of the six-degree-of-freedom series-connected mechanical arm 6 through a support plate 55.

Optionally, the resectoscope holding device 5 includes a pushing block 51, a sliding block 52, a motor 53 and a synchronous belt 54.

The pushing block 51 is fixed to the cutting loop 41, the sliding block 52 is fixed to the pushing block 51, and the synchronous belt 54 is fixed to sliding block 52. When the motor 53 rotates, the synchronous belt 54 is driven to move, so that the motor 53 drives the cutting loop 41 to move forward and backward along the axis of the resectoscope. The motor 53 may be a direct current servo motor. The workstation 3 acquires an encoder pulse of the direct current servo motor in real time for calculating a real-time position.

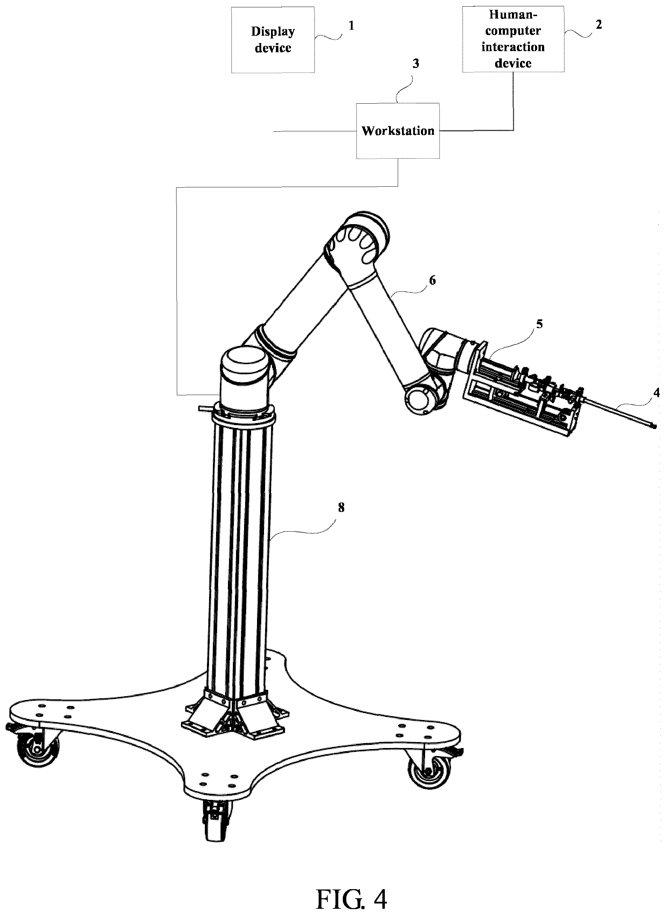

Optionally, on the basis of the above technical solutions, as shown in FIG. 4, the robot-assisted system for transurethral surgery may further include a mobile platform 8.

The mobile platform 8 is fixed to a base of the six-degree-of-freedom series-connected mechanical arm 6, and is configured to support the six-degree-of-freedom series-connected mechanical arm 6 and drive the six-degree-of-freedom series-connected mechanical arm 6 to move on the ground.

Exemplarily, the mobile platform 8 may be configured with 4 universal wheels of which at least two universal wheels are disposed with a locking mechanism, which may facilitate moving the six-degree-of-freedom series-connected mechanical arm 6, the resectoscope holding device 5 connected to the six-degree-of-freedom series-connected mechanical arm 6 and the resectoscope 4 clamped to the resectoscope holding device 5, and locking the universal wheels after arriving at the specified position.

Preferably, in the robot-assisted system for transurethral surgery, the technical solutions in the embodiment of the present disclosure may also implement automatic positioning for lesions in combination with resectoscope images in addition to implement the control of the six-degree-of-freedom series-connected mechanical arm 6 by the human-computer interaction device 2. The surgeon selects an area represented by a certain point P in the resectoscope image as the resection area. The workstation 3 performs calculation, and controls the six-degree-of-freedom series-connected mechanical arm 6 rotates around a certain axis in the space. The final effect is to achieve that a lowest point of the cutting loop 41moves to point P. In the above operation process, the surgeon does not need to adjust the various joints of the six-degree-of-freedom series-connected mechanical arm 6 one by one, and only needs to select a specific position. Movement commands required by the six-degree-of-freedom series-connected mechanical arm 6 are transmitted to a controller of the six-degree-of-freedom series-connected mechanical arm 6 to be executed by a communication port (such as a TCP/IP Ethernet interface) after the workstation acquires data and calculates the data.

Generally, when the surgery starts, the surgeon enables the electrified wire loop to aim at the surgical area according to the above manner. Then the surgeon selects the DC servo motor on which a control object is located by the function key on the human-computer interaction device 2. The surgeon only needs to push/retract the operating rod in a first preset axis direction to control forward/backward rotation of the DC servo motor. Implementation process is as follows: the workstation 3 reads data of the operating rod, the data is processed and transformed into position information about the electrified wire loop, and transmitted to a microprocessor (such as a single-chip) of the resectoscope holding device 5 through the communication port (such as a USB). After the position information is processed by the microprocessor, PWM waves are generated and transformed into a voltage by a driver to control the DC servo motor to rotate. Meanwhile, the microprocessor acquires the encoder pulse of the DC servo motor in real time for calculating the real-time position. And compared the real-time position with a target position transmitted by the workstation 3, a delta is used for generating PWM to form closed loop control. The pushing block and the sliding block controlling the stretching and withdrawing of the cutting loop 41 on the resectoscope holding device 5 are fixed. The DC servo motor drives the synchronous belt to rotate, thereby driving the sliding block fixed to the synchronous belt to translate, and finally achieves the stretching and withdrawing movement of the cutting loop 41. During the surgery, the surgeon pushes forward the operating rod in the first preset axis direction, the cutting loop 41 stretches out to contact the pathological tissues. Then the surgeon retreats the operating rod in a negative direction of the first preset axis, in the process of the retreat of the cutting loop 41, the current intensity of the cutting loop 41 is controlled by an energy platform, thereby cutting the pathological tissues off.

It should be noted that the above are only preferred embodiments of the present application and the technical principles used therein. It will be understood by those skilled in the art that the present application is not limited to the specific embodiments described herein. Those skilled in the art can make various apparent modifications, adaptations and substitutions without departing from the scope of the present application. Therefore, while the present application has been described in detail via the above-mentioned embodiments, the present application is not limited to the above-mentioned embodiments and may include more other equivalent embodiments without departing from the concept of the present application. The scope of the present application is determined by the scope of the appended claims.

## Claims

1. A robot-assisted system for transurethral surgery, comprising:
a workstation (3);
a display device (1), wherein the display device (1) is communicatively connected to the workstation (3), and configured to receive display data transmitted from the workstation (3) and display images to a surgeon based on the display data;
a human-computer interaction device (2), wherein the human-computer interaction device (2) is communicatively connected to the workstation (3), and configured to acquire operation instructions input by the surgeon and transmit the operation instructions to the workstation (3);
a resectoscope (4) comprising a cutting loop (41), wherein the resectoscope (4) is communicatively connected to the workstation (3), and configured to transmit acquired image information to the workstation (3) and control a current intensity of the cutting loop (41) according to a first control signal received from the workstation (3);
a resectoscope holding device (5), wherein the resectoscope holding device (5) is communicatively connected to the workstation (3), and configured to hold the resectoscope (4) and drive the cutting loop (41) to move forward and backward along an axis of the resectoscope (4) according to a second control signal received from the workstation (3);
a six-degree-of-freedom series-connected mechanical arm (6), wherein the six-degree-of-freedom series-connected mechanical arm (6) is communicatively connected to the workstation (3) and fixed to the resectoscope holding device (5), and the six-degree-of-freedom series-connected mechanical arm (6) is configured to drive, according to a third control signal received from the workstation (3), the resectoscope holding device (5) to move forward and backward along the axis of the resectoscope (4), rotate around the central axis of the resectoscope (4) and rotate around a preset point on the central axis of the resectoscope (4);
wherein the workstation (3) is configured to: generate the display data according to the image information acquired from the resectoscope (4) and transmit the display data to the display device (1); generate the first control signal according to the operation instructions acquired from the human-computer interaction device (2) and transmit the first control signal to the resectoscope (4); and/or generate the second control signal and transmit the second control signal to the resectoscope holding device (5); and/or generate the third control signal and transmit the third control signal to the six-degree-of-freedom series-connected mechanical arm (6);
wherein the workstation (3) is configured to: based on an area represented by a certain point P in a resectoscope image being selected as a resection area, perform calculation, and control the six-degree-of-freedom series-connected mechanical arm (6) to rotate around a certain axis in space to achieve that a lowest point of the cutting loop (41) moves to the point P.

2. The robot-assisted system for transurethral surgery according to claim 1, wherein the human-computer interaction device (2) comprises a three-degree-of-freedom operating rod or a three-degree-of-freedom mouse, and the human-computer interaction device (2) further comprises six first function keys and a second function key.

3. The robot-assisted system for transurethral surgery according to claim 2 wherein the six first function keys and six joints of the six-degree-of-freedom series-connected mechanical arm (6) are in a one-to-one correspondence, and each of the six first function keys is used for activating or exiting a control mode of one of the six joints corresponding to the first function key according to input operations of the surgeon; the second function key is used for acquiring a key travel signal generated by pressing the second function key by the surgeon so that the workstation (3) generates the first control signal according to the key travel signal.

4. The robot-assisted system for transurethral surgery according to any preceding claim, further comprising:
a force sensor, which is communicatively connected to the workstation (3) and disposed at the junction of the resectoscope holding device (5) and the six-degree-of-freedom series-connected mechanical arm (6), wherein the force sensor is configured to measure a contacting force between the resectoscope (4) and an object which is contact with the resectoscope (4), and transmit a signal about the contacting force to the workstation (3).

5. The robot-assisted system for transurethral surgery according to claim 4, wherein the workstation (3) is further configured to control the resectoscope (4), the resectoscope holding device (5) and the six-degree-of-freedom series-connected mechanical arm (6) to stop working in response to determining that the signal about the contacting force acquired from the force sensor is greater than a preset value.

6. The robot-assisted system for transurethral surgery according to any preceding claim, wherein the resectoscope holding device (5) is screwed to a flange at an end joint of the six-degree-of-freedom series-connected mechanical arm (6) through a support plate.

7. The robot-assisted system for transurethral surgery according to any preceding claim, wherein the resectoscope holding device (5) comprises a pushing block (51), a sliding block (52), a motor (53) and a synchronous belt (54); and
the pushing block (51) is fixed to the cutting loop (41), the sliding block (52) is fixed to the pushing block (51), and the synchronous belt (54) is fixed to sliding block (52), the synchronous belt (54) is driven to move when the motor (53) rotates, so that the motor (53) drives the cutting loop (41) to move forward and backward along the axis of the resectoscope (4).

8. The robot-assisted system for transurethral surgery according to claim 7, wherein the motor (53) is a direct current servo motor.

9. The robot-assisted system for transurethral surgery according to any one of claims 1 to 8, further comprising:
a mobile platform (8), which is fixed to a base of the six-degree-of-freedom series-connected mechanical arm (6), and is configured to support the six-degree-of-freedom series-connected mechanical arm (6) and drive the six-degree-of-freedom series-connected mechanical arm (6) to move on the ground.

## Patentansprüche

1. Robotergestütztes System für die transurethrale Chirurgie, umfassend:
eine Workstation (3);
eine Anzeigevorrichtung (1), wobei die Anzeigevorrichtung (1) kommunikationsfähig mit der Workstation (3) verbunden ist und gestaltet ist für den Empfang von Anzeigedaten, die von der Workstation (3) übermittelt werden, und zum Anzeigen von Bildern an einen Chirurgen auf der Basis der Anzeigedaten;
eine Mensch-Computer-Interaktionsvorrichtung (2), wobei die Mensch-Computer-Interaktionsvorrichtung (2) kommunikationsfähig mit der Workstation (3) verbunden ist und so gestaltet ist, dass sie von einem Chirurgen eingegebene Betriebsanweisungen erfasst und die Betriebsanweisungen an die Workstation (3) übermittelt;
ein Resektoskop (4), das eine Schneideschleife (41) umfasst, wobei das Resektoskop (4) kommunikationsfähig mit der Workstation (3) verbunden ist und so gestaltet ist, dass es erfasste Bildinformationen an die Workstation (3) übermittelt und eine Stromintensität der Schneideschleife (41) gemäß einem von der Workstation (3) empfangenen ersten Steuersignal steuert;
eine Resektoskop-Haltevorrichtung (5), wobei die Resektoskop-Haltevorrichtung (5) kommunikationsfähig mit der Workstation (3) verbunden ist und so gestaltet ist, dass sie das Resektoskop (4) hält und die Schneideschleife (41) so steuert, dass sich diese entlang einer Achse des Resektoskops (4) gemäß einem von der Workstation (3) empfangenen zweiten Steuersignal vor und zurück bewegt;
einen in Reihe verbundenen mechanischen Arm (6) mit sechs Freiheitsgraden, wobei der in Reihe verbundene mechanische Arm (6) mit sechs Freiheitsgraden kommunikationsfähig mit der Workstation (3) verbunden ist und an der Resektoskop-Haltevorrichtung (5) befestigt ist, und wobei der in Reihe verbundene mechanische Arm (6) mit sechs Freiheitsgraden so gestaltet ist, dass er die Resektoskop-Haltevorrichtung (5) gemäß einem von der Workstation (3) empfangenen dritten Steuersignal so steuert, dass sich diese entlang einer Achse des Resektoskops (4) vor und zurück bewegt, dass sie sich um die zentrale Achse des Resektoskops (4) dreht und dass sie sich um einen vorab festgelegten Punkt an der zentralen Achse des Resektoskops (4) dreht;
wobei die Workstation (3) so gestaltet ist, dass sie die Anzeigedaten gemäß den von dem Resektoskop (4) erfassten Bildinformationen erzeugt und die Anzeigedaten an die Anzeigevorrichtung (1) übermittelt; dass sie das erste Steuersignal gemäß den von der Mensch-Computer-Interaktionsvorrichtung (2) erfassten Betriebsanweisungen erzeugt und das erste Steuersignal an das Resektoskop (4) übermittelt; und/oder dass sie das zweite Steuersignal erzeugt und das zweite Steuersignal an die Resektoskop-Haltevorrichtung (5) übermittelt; und/oder dass sie das dritte Steuersignal erzeugt und das dritte Steuersignal an den in Reihe verbundenen mechanischen Arm (6) mit sechs Freiheitsgraden übermittelt;
wobei die Workstation (3) so gestaltet ist, dass sie: auf der Basis eines durch einen bestimmten Punkt P in einem Resektoskopbild als Resektionsbereich ausgewählten Bereich Berechnungen ausführt und den in Reihe verbundenen mechanischen Arm (6) mit sechs Freiheitsgraden so steuert, dass sich dieser um eine bestimmte Achse im Raum dreht, um zu erreichen, dass sich ein unterster Punkt der Schneideschleife (41) zu dem Punkt P bewegt.

2. Robotergestütztes System für die transurethrale Chirurgie nach Anspruch 1, wobei die Mensch-Computer-Interaktionsvorrichtung (2) einen Betriebsstab mit drei Freiheitsgraden oder eine Maus mit drei Freiheitsgraden umfasst, und wobei die Mensch-Computer-Interaktionsvorrichtung (2) ferner sechs erste Funktionstasten und eine zweite Funktionstaste umfasst.

3. Robotergestütztes System für die transurethrale Chirurgie nach Anspruch 2, wobei sich die sechs ersten Funktionstasten und sechs Gelenke des in Reihe verbundenen mechanischen Arms (6) mit sechs Freiheitsgraden Eins-zu-Eins entsprechen, und wobei jede der sechs ersten Funktionstasten verwendet wird, um eine Steuermodus eines der sechs Gelenke entsprechend der ersten Funktionstaste gemäß eingegebenen Anweisungen durch den Chirurgen zu aktivieren oder zu verlassen; wobei die zweite Funktionstaste zur Erfassung eines Tastenbewegungssignals verwendet wird, das erzeugt wird durch Drücken der zweiten Funktionstaste durch den Chirurgen, so dass die Workstation (3) das erste Steuersignal gemäß dem Tastenbewegungssignal erzeugt.

4. Robotergestütztes System für die transurethrale Chirurgie nach einem der vorstehenden Ansprüche, das ferner folgendes umfasst:
einen Kraftsensor, der kommunikationsfähig mit der Workstation (3) verbunden ist und sich an der Verbindungsstelle der Resektoskop-Haltevorrichtung (5) und des in Reihe verbundenen mechanischen Arms (6) mit sechs Freiheitsgraden befindet, wobei der Kraftsensor so gestaltet ist, dass er eine Kontaktkraft zwischen dem Resektoskop (4) und einem Objekt misst, das sich in Kontakt mit dem Resektoskop (4) befindet, und um ein Signal über die Kontaktkraft an die Workstation (3) zu übermitteln.

5. Robotergestütztes System für die transurethrale Chirurgie nach Anspruch 4, wobei die Workstation (3) ferner so gestaltet ist, dass sie das Resektoskop (4), die Resektoskop-Haltevorrichtung (5) und den in Reihe verbundenen mechanischen Arm (6) mit sechs Freiheitsgraden so steuert, dass der Betrieb angehalten wird als Reaktion auf die Bestimmung, dass das Signal über die von dem Kraftsensor erfasste Kontaktkraft größer ist als ein vorher festgelegter Wert.

6. Robotergestütztes System für die transurethrale Chirurgie nach einem der vorstehenden Ansprüche, wobei die Resektoskop-Haltevorrichtung (5) an einem Endgelenk des in Reihe verbundenen mechanischen Arms (6) mit sechs Freiheitsgraden durch eine Trägerplatte an einen Flansch geschraubt ist.

7. Robotergestütztes System für die transurethrale Chirurgie nach einem der vorstehenden Ansprüche, wobei die Resektoskop-Haltevorrichtung (5) einen Schiebeblock (51), einen Gleitblock (52), einen Motor (53) und einen Zahnriemen (54) umfasst; und
wobei der Schiebeblock (51) an der Schneideschleife (41) befestigt ist, wobei der Gleitblock (52) an dem Schiebeblock (51) befestigt ist, und wobei der Zahnriemen (54) an dem Gleitblock (52) befestigt ist, wobei der Zahnriemen (54) so angetrieben wird, dass er sich bewegt, wenn sich der Motor (53) dreht, so dass der Motor (53) die Schneideschleife (41) so antreibt, dass sich diese entlang der Achse des Resektoskops (4) vor und zurück bewegt.

8. Robotergestütztes System für die transurethrale Chirurgie nach Anspruch 7, wobei der Motor (53) ein Gleichstrom-Servomotor ist.

9. Robotergestütztes System für die transurethrale Chirurgie nach einem der Ansprüche 1 bis 8, ferner umfassend:
eine mobile Plattform (8), die an einer Basis des in Reihe verbundenen mechanischen Arms (6) mit sechs Freiheitsgraden befestigt ist und so gestaltet ist, dass sie den in Reihe verbundenen mechanischen Arm (6) mit sechs Freiheitsgraden trägt und den in Reihe verbundenen mechanischen Arm (6) mit sechs Freiheitsgraden so antreibt, dass sich dieser über dem Boden bewegt.

## Revendications

1. Système assisté par un robot pour la chirurgie transurétrale, comprenant :
un poste de travail (3) ;
un dispositif d'affichage (1), le dispositif d'affichage (1) étant connecté en communication au poste de travail (3), et conçu pour recevoir les données d'affichage transmises par le poste de travail (3) et afficher des images à un chirurgien sur la base des données d'affichage ;
un dispositif d'interaction homme-machine (2), le dispositif d'interaction homme-machine (2) étant connecté en communication au poste de travail (3), et conçu pour acquérir des instructions d'opération entrées par le chirurgien et transmettre les instructions d'opération au poste de travail (3) ;
un résectoscope (4) comprenant une boucle de coupe (41), le résectoscope (4) étant connecté en communication au poste de travail (3), et conçu pour transmettre les informations d'image acquises au poste de travail (3) et commander une intensité de courant de la boucle de coupe (41) selon un premier signal de commande reçu du poste de travail (3) ;
un dispositif de maintien de résectoscope (5), le dispositif de maintien de résectoscope (5) étant connecté en communication au poste de travail (3), et conçu pour maintenir le résectoscope (4) et entraîner la boucle de coupe (41) pour qu'elle se déplace vers l'avant et vers l'arrière le long d'un axe du résectoscope (4) selon un deuxième signal de commande reçu du poste de travail (3) ;
un bras mécanique (6) connecté en série à six degrés de liberté, le bras mécanique (6) connecté en série à six degrés de liberté étant connecté en communication au poste de travail (3) et fixé au dispositif de maintien de résectoscope (5), et le bras mécanique (6) connecté en série à six degrés de liberté étant conçu pour entraîner, selon un troisième signal de commande reçu du poste de travail (3), le dispositif de maintien de résectoscope (5) pour qu'il se déplace vers l'avant et vers l'arrière le long de l'axe du résectoscope (4), tourne autour de l'axe central du résectoscope (4) et tourne autour d'un point préréglé sur l'axe central du résectoscope (4) ;
le poste de travail (3) étant conçu pour : générer les données d'affichage en fonction des informations d'image acquises du résectoscope (4) et transmettre les données d'affichage au dispositif d'affichage (1) ; générer le premier signal de commande en fonction des instructions d'opération acquises par le dispositif d'interaction homme-machine (2) et transmettre le premier signal de commande au résectoscope (4) ; et/ou générer le deuxième signal de commande et transmettre le deuxième signal de commande au dispositif de maintien de résectoscope (5) ; et/ou générer le troisième signal de commande et transmettre le troisième signal de commande au bras mécanique (6) connecté en série à six degrés de liberté ;
le poste de travail (3) étant conçu pour : sur la base d'une zone représentée par un certain point P dans une image de résectoscope sélectionnée comme zone de résection, effectuer des calculs, et commander le bras mécanique (6) connecté en série à six degrés de liberté pour qu'il tourne autour d'un certain axe dans l'espace afin qu'un point le plus bas de la boucle de coupe (41) se déplace au point P.

2. Système assisté par un robot pour la chirurgie transurétrale selon la revendication 1, le dispositif d'interaction homme-machine (2) comprenant une tige de commande à trois degrés de liberté ou une souris à trois degrés de liberté, et le dispositif d'interaction homme-machine (2) comprenant en outre six premières touches de fonction et une seconde touche de fonction.

3. Système assisté par un robot pour la chirurgie transurétrale selon la revendication 2, les six premières touches de fonction et les six articulations du bras mécanique (6) connecté en série à six degrés de liberté étant en correspondance biunivoque, et chacune des six premières touches de fonction étant utilisée pour activer ou désactiver un mode de commande de l'une des six articulations correspondant à la première touche de fonction selon les opérations d'entrée du chirurgien ; la seconde touche de fonction étant utilisée pour acquérir un signal de déplacement de touche généré par le chirurgien en appuyant sur la seconde touche de fonction, de sorte que le poste de travail (3) génère le premier signal de commande en fonction du signal de déplacement de touche.

4. Système assisté par un robot pour la chirurgie transurétrale selon l'une quelconque des revendications précédentes, comprenant en outre :
un capteur de force, qui est connecté en communication au poste de travail (3) et disposé à la jonction du dispositif de maintien de résectoscope (5) et du bras mécanique (6) connecté en série à six degrés de liberté, le capteur de force étant conçu pour mesurer une force de contact entre le résectoscope (4) et un objet qui est en contact avec le résectoscope (4), et pour transmettre un signal concernant la force de contact au poste de travail (3).

5. Système assisté par un robot pour la chirurgie transurétrale selon la revendication 4, le poste de travail (3) étant en outre conçu pour commander le résectoscope (4), le dispositif de maintien de résectoscope (5) et le bras mécanique (6) connecté en série à six degrés de liberté pour arrêter de fonctionner en réponse à la détermination du fait que le signal concernant la force de contact acquis par le capteur de force est supérieur à une valeur prédéfinie.

6. Système assisté par un robot pour la chirurgie transurétrale selon l'une quelconque des revendications précédentes, le dispositif de maintien de résectoscope (5) étant vissé à une bride au niveau d'une articulation d'extrémité du bras mécanique (6) connecté en série à six degrés de liberté par l'intermédiaire d'une plaque de support.

7. Système assisté par un robot pour la chirurgie transurétrale selon l'une quelconque des revendications précédentes, le dispositif de maintien de résectoscope (5) comprenant un bloc de poussée (51), un bloc coulissant (52), un moteur (53) et une courroie synchrone (54) ; et
le bloc de poussée (51) étant fixé à la boucle de coupe (41), le bloc coulissant (52) étant fixé au bloc de poussée (51), et la courroie synchrone (54) étant fixée au bloc coulissant (52), la courroie synchrone (54) étant entraînée pour se déplacer lorsque le moteur (53) tourne, de sorte que le moteur (53) entraîne la boucle de coupe (41) pour qu'elle se déplace vers l'avant et vers l'arrière le long de l'axe du résectoscope (4).

8. Système assisté par un robot pour la chirurgie transurétrale selon la revendication 7, le moteur (53) étant un servomoteur à courant continu.

9. Système assisté par un robot pour la chirurgie transurétrale selon l'une quelconque des revendications 1 à 8, comprenant en outre :
une plate-forme mobile (8), qui est fixée à une base du bras mécanique (6) connecté en série à six degrés de liberté, et qui est conçue pour supporter le bras mécanique (6) connecté en série à six degrés de liberté et pour entraîner le bras mécanique (6) connecté en série à six degrés de liberté pour qu'il se déplace sur le sol.
